# EUROPEAN PATENT SPECIFICATION

(11) **EP 3 310 935 B1**
(45) Date of publication and mention of the grant of the patent: **06.12.2023**
(21) Application number: 16815096.9
(22) Date of filing: 17.06.2016
(51) Int. Cl.: C12M 1/34, G01N 21/64, G06T 7/00, B01L 3/00, B01L 7/00

(54) **DEVICE FOR ANALYZING A FLUID SAMPLE AND USE OF TEST CARD WITH SAME**
VORRICHTUNG ZUR ANALYSE EINER FLÜSSIGKEITSPROBE UND VERWENDUNG EINER TESTKARTE DAMIT
DISPOSITIF D'ANALYSE D'UN ÉCHANTILLON DE FLUIDE ET UTILISATION D'UNE CARTE DE TEST AVEC CELUI-CI

(30) Priority: 22.06.2015 US 201562182992 P; 01.07.2015 US 201562187471 P
(43) Date of publication of application: 25.04.2018
(60) Divisional application: 23161706.9
(73) Proprietor: FluxErgy, Inc., Irvine, CA 92618 (US)
(72) Inventor: REVILLA, Ryan, Huntington Beach, CA 92646 (US); HELTSLEY, Roy, Foothill Ranch, CA 92610 (US); LEE, Steve, Hoe, Glendale, CA 91208 (US); IZADI KHARAZI, Farzad, Encinitas, CA 92024 (US); PATEL, Tej, Aliso Viejo, CA 92656 (US)
(74) Representative: Murgitroyd & Company
(86) International application number: PCT/US2016/038152
(87) International publication number: WO 2016/209734

(56) References cited:
- WO-A1-93/22053
- WO-A1-93/22054
- WO-A2-2014/144548
- US-A1- 2006 094 028
- US-A1- 2009 053 726
- US-A1- 2009 143 233
- US-A1- 2011 206 545
- US-A1- 2011 315 559
- US-A1- 2012 052 560
- US-A1- 2015 352 548

## Description

### FIELD OF THE DISCLOSURE

The present disclosure relates generally to a portable device for a fluid sample assay, and more specifically to a portable device that accepts disposable test cards to quickly and conveniently analyze a plurality of polymerase chain reactions.

### BACKGROUND OF THE DISCLOSURE

Point-of-care (POC) *in vitro* diagnostics tests (IVDT) have traditionally had two major categories, nucleic acid amplification tests (NAAT) or immunoassay-based tests. The former directly detects the pathogen's DNA or RNA, while the latter detects antibodies or antigens generated by the immune system response to the pathogen.

Current POC diagnostic immunoassays lack the high sensitivity and specificity of nucleic acid amplification methods. This becomes more pronounced during the initial stages of infection, often within 168 hours. Taking the case of Dengue virus in whole blood, immunoglobulin M (IgM) and immunoglobulin G (IgG) remain undetectable in the majority of patients until 5 and 10 days post-infection, respectively, whereas nucleic acid can be found as early as 0 to 7 days. Moreover, many immunoassay tests are unable to detect infectious agents until 3 months after the initial onset of the infection. This delay is due to the time it takes for the body's immune system to respond to an infection.

POC diagnostic assays developed utilizing NAATs have very high sensitivities and specificities, matching those of currently accepted laboratory tests. The primary mechanism of NAAT based systems is to directly detect an infectious agent's nucleic acid, lending to the test's ability to detect diseases within the first few days of the onset of infection. In addition, by careful primer design, NAATs also have the ability to have very high specificity and sensitivity compared to immunoassay based testing. The largest drawback of NAATs compared to immunoassay-based tests is the complicated equipment and/or processes required to prepare a sample for testing.

US 2012/052560 of Ivor Knight et al describes systems and methods for the rapid serial processing of multiple nucleic acid assays. The document provides for the real time processing of nucleic acid during polymerase chain reaction (PCR) and thermal melt applications.

### SUMMARY OF THE DISCLOSURE

The present invention relates to a device for performing an assay on a fluid sample in accordance with Claim 1 herein.

In an example embodiment, the device includes a user interface, wherein the controller is configured to output the resulting analysis to the user interface to be displayed to a user of the device.

In an example embodiment, the device includes a light source configured to illuminate at least a portion of the microchannel when the test card is received within the slot.

In an example embodiment, the image includes at least one of: (i) a plurality of still images recorded by the camera imaging device over a period of time; or (ii) a video image recorded by the camera imaging device over the period of time.

In an example embodiment, the outlet port is located on an upper surface of the test card, and the vacuum source is configured to align with the outlet port on the upper surface of the test card.

In an example embodiment, the device includes a button configured to align the vacuum source with the outlet port on the upper surface of the test card.

In an example embodiment, the test card includes an analysis port located on an upper surface of the test card, and the camera imaging system is configured to align with the analysis port on the upper surface of the test card.

In an example embodiment, the at least one electrical contact is located on a bottom surface of the test card, and the electrical contact device is configured to align with the at least one electrical contact on the bottom surface of the test card.

The invention further relates to a method of using the device of any of claims 1-12 in accordance with claim 13 herein.

### BRIEF DESCRIPTION OF THE DRAWINGS

Embodiments of the present disclosure will now be explained in further detail by way of example only with reference to the accompanying figures, in which:
FIG. 1 is a top perspective view of an example embodiment of an assay device according to the present disclosure;
FIG. 2 is a cross-sectional view of the assay device of FIG. 1;
FIG. 3 is a top perspective view of an example embodiment of a test card for use with the assay device of FIG. 1 in a diagnostic system according to the present disclosure;
FIG. 4 is a cross-sectional view of the test card of FIG. 3;
FIG. 5 is a bottom perspective view of the test card of FIG. 3;
FIG. 6A is a top view of the schematics of the electrical contacts of the test card of FIG. 3;
FIG. 6B is a top view of the schematics of the electrical contacts of the test card of FIG. 3 aligned with the microchannel of the test card;
FIG. 7 is a top perspective view of the assay device of FIG. 1 with the test card of FIG. 3 inserted therein;
FIG. 8 is a cross-sectional view of the assay device and test card shown in FIG. 7; and
FIG. 9 is a top perspective view of an example embodiment of an electrical contact device according to the present disclosure;
FIG. 10 illustrates an example embodiment of a control method that can be used to analyze a fluid sample according to the present disclosure; and
FIG. 11 illustrates an example embodiment of a controller that can perform the method of FIG. 10.

### DETAILED DESCRIPTION

Before describing in detail the illustrative system and method of the present disclosure, it should be understood and appreciated herein that the present disclosure relates to a rapid, high sensitivity and high specificity, low complexity, diagnostic system 1 using nucleic acid amplification capable of operating in low resource settings. The system described herein is configured, for example, to cause and analyze a polymerase chain reaction (PCR), particularly in the early stages of infection, using a low-cost microfluidic platform employing a PCR with a modified DNA polymerase.

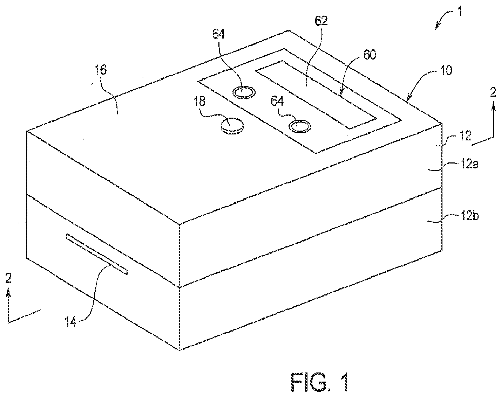

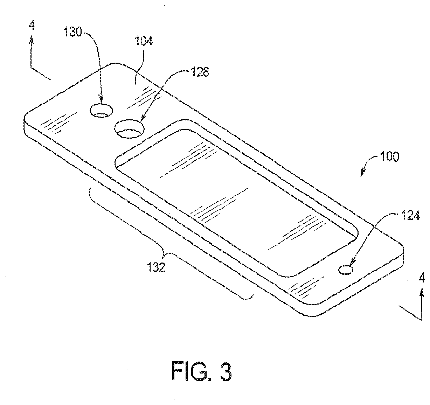

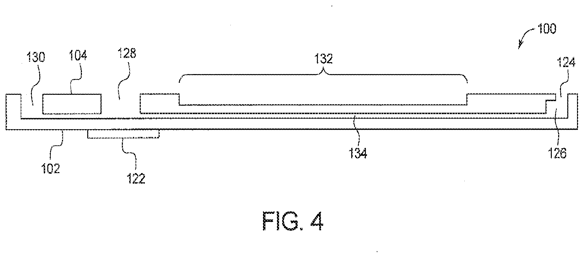

FIG. 1 illustrates an example embodiment of a point-of-care diagnostic system 1 according to the present disclosure. As illustrated, diagnostic system 1 includes an assay device 10 with a housing 12 having a slot 14 to receive a test card 100 (FIGS. 3 to 5), which is an inexpensive disposable test card that can be used with device 10 and then discarded. In use, and as explained in more detail below, a fluid sample can be injected into test card 100, and then test card 100 can be inserted into slot 14 so that device 10 can power test card 100 and analyze a fluid sample within test card 100 without further action by the user. An advantage of diagnostic system 1 is that a plurality of test cards 100 can be used with a single assay device 10 to inexpensively perform a plurality of assays on a single fluid sample or on a plurality of fluid samples. In an embodiment, the assay includes at least one of a polymerase chain reaction (PCR) assay, a flow cytometry assay, or an enzyme-linked immunosorbent assay (ELISA). In an embodiment, the test card 100 is configured to receive about 10 µL of whole blood, the equivalent of a drop of blood obtained from a finger stick. In another embodiment, the fluid sample can be serum, urine, saliva, tears and/or the like.

Housing 12 of assay device 10 is an outer casing for a plurality of electrical components configured to move and analyze the fluid sample within test card 100 when test card 100 is inserted into slot 14. In the illustrated embodiment, housing 12 is formed of a top portion 12a and a bottom portion 12b that can be mated to enclose the plurality of electrical components. Housing 10 can be formed, for example, from a variety of billet or extruded metals and plastics, as well as injection molded plastics. In an embodiment, housing 10 is formed of 6061-T6 Aluminum. As illustrated in more detail below, slot 14 allows test card 100 to be inserted horizontally into housing 12, so that one or more microchannels 134 within test card 100 are aligned horizontally when one or more assays occur within device 10.

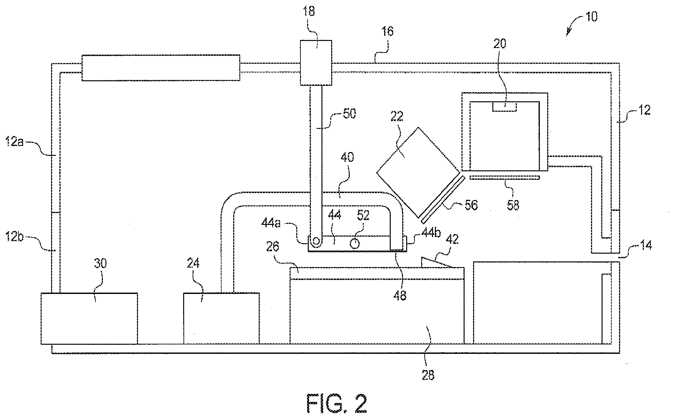

FIG. 2 illustrates a cross sectional view of assay device 10 showing the configuration of the elements located within housing 12, before test card 100 has been inserted into slot 14. Structural elements such as screws and brackets holding the various elements in place in FIG. 2 have been omitted for simplicity. Electrical wiring has also been omitted for simplicity. It should further be understood by those of ordinary skill in the art that the elements located within housing 12 can be arranged in various configurations and still function as described below.

As illustrated in FIG. 2, assay device 10 includes a camera imaging device 20, a light source 22, a fluid actuation source 24, an electrical contact device 26, a controller 28 and a power source 30. In use, and as explained in more detail below, camera imaging device 20, light source 22, fluid actuation source 24, and electrical contact device 26 align with elements of test card 100 as test card 100 is inserted horizontally into slot 14. Once test card 100 is fully inserted into slot 14, controller 28 controls each of camera imaging device 20, light source 22, fluid actuation source 24, electrical contact device 26 and power source 30 to pull a fluid sample through test card 100, heat the fluid sample if necessary for the assay, and analyze the fluid sample.

FIGS. 3 to 5 illustrate an example embodiment of a test card 100 configured to be inserted into slot 14. Example embodiments of test card 100 are described in more detail in U.S. Application No. 15/185,661, entitled "Test Card for Assay and Method of Manufacturing Same", filed concurrently herewith under Attorney Docket No. 1958928-00007. Those of ordinary skill in the art will recognize other configurations of test cards 100 that can be used with device 10.

As illustrated, test card 100 includes an inlet port 124, a mixing chamber 126, a capture port 128, an outlet port 130, and a fluid microchannel 134. In use, a liquid sample can be injected into inlet port 124 and mix with a reagent in mixing chamber 126, and then test card 100 can be placed into slot 14 of assay device 10. As explained in more detail below, once test card 100 has been placed within assay device 10, the fluid sample can be pulled though fluid microchannel 134, so that the fluid sample can be analyzed through an analysis port 132 on an upper surface 104 of test card 100. Test card 100 also includes electrical contacts 122 on a bottom surface 102 thereof, which enable electrodes adjacent to fluid microchannel 134 to be controlled to heat fluid within fluid microchannel 134, track fluid flow through fluid microchannel 134, and/or measure properties of fluid within microchannel 134 such as the quantity of the chemical species in the fluid sample.

As explained in more detail in U.S. Application No. 15/185,661, Attorney Docket No. 1958928-00007, fluid microchannel 134 of test card 100 includes a target zone 166 and optionally a first fluid detection zone 150 upstream of target zone 166 and/or a second fluid detection zone 154 downstream of target zone 166. Target zone 166, first fluid detection zone 150 and second fluid detection zone 154 include respective electrodes 160, 162, 164, which, for example, are screen printed on test card 100 using a dielectric ink. When a current is applied to electrodes 162 of target zone 166, the current across electrodes 162 can heat the fluid sample within target zone 166 to cause a PCR. Electrodes 160 and 164 of first fluid detection zone 150 and second fluid detection zone 154, respectively, form capacitance sensors that can be used to detect whether or not the fluid sample is present within first fluid detection zone 50 and/or second fluid detection zone 154, as the dielectric constant of the capacitance sensors differs considerably when there is liquid in the microchannel. In another embodiment of an ion selective electrode test card, the electrodes can be used, for example, to measure the potential difference between two reactive electrodes within test card 100's microchannels 134 allowing for the measurement of various blood analytes such as sodium levels.

In an alternative embodiment, any of electrodes 160, 162, 164 can be used for a specific chemical species detection. When a fluid sample is present within microchannel 134, the dielectric constant across the electrodes will change depending on the quantity of the chemical species in the fluid sample. If microchannel 134 is located adjacent to a capacitor, then the amount of chemical species can be measured based on the measured capacitance across the electrodes. For example, during a PCR, there is initially very little DNA present, so there will be a very small dielectric constant. As the PCR progresses, the dielectric value will change as more DNA is produced. Any of electrodes 160, 162, 164 can therefore be used to measure a specific chemical species by detecting the dielectric value of the fluid sample.

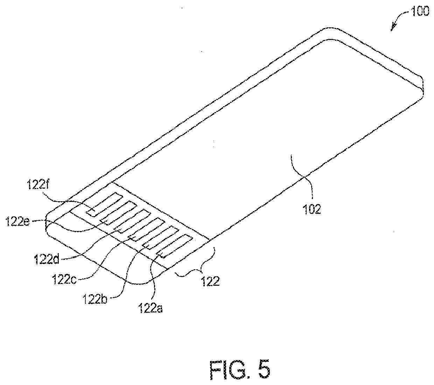

As illustrated in FIG. 5, the bottom surface 102 of test card 100 includes a plurality of electrical contacts 122, individually labeled as electrical contacts 122a, 122b, 122c, 122d, 122e and 122f. The electrical contacts 122 are in electrical contact with electrodes 160, 162, 164, so that a current applied to electrical contacts 122 can transmitted to electrodes 164 to cause a PCR and/or transmitted to electrodes 160 and 162 to monitor fluid within fluid microchannel 134. Electrical contacts 122 can also be multiplexed, allowing for both the application and measurement of AC and DC voltage and current.

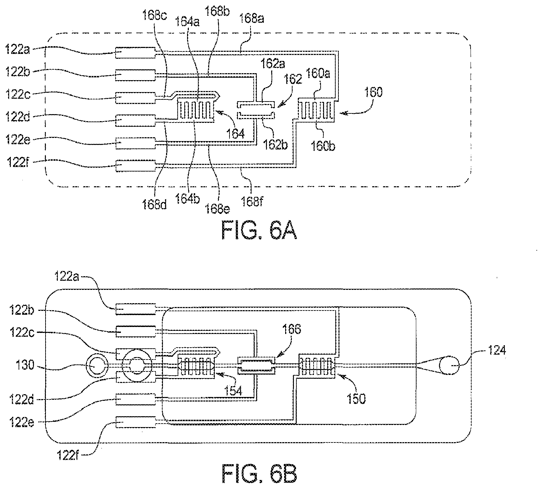

FIGS. 6A and 6B illustrate an example embodiment of the specific electrical connections of electrical contacts 122a, 122b, 122c, 122d, 122e and 122f. FIG. 6A shows the electrical connections without fluid microchannel 134, and FIG. 6B shows the electrical connections aligned with fluid microchannel.

In the illustrated embodiment, electrical contact 122a is electrically connected via electrical line 168a to a first plurality of electrodes 160a of electrodes 160 of first fluid detection zone 150, and electrical contact 122f is electrically connected via electrical line 168f to a second plurality of electrodes 160b of electrodes 160 of first fluid detection zone 150. Electrical contact 122b is electrically connected via electrical line 168b to a first electrode 162a of electrodes 162 of target zone 166, and electrical contact 122e is electrically connected via electrical line 168e to a second electrode 162b of electrodes 162 of target zone 166. Electrical contact 122c is electrically connected via electrical line 168c to a first plurality of electrodes 164a of electrodes 164 of second fluid detection zone 154, and electrical contact 122d is electrically connected via electrical line 168d to a second plurality of electrodes 164b of electrodes 164 of second fluid detection zone 154. Operation of target zone 166, first fluid detection zone 150 and second fluid detection zone 154 by applying a current to one or more of electrical contacts 122a, 122b, 122c, 122d, 122e and 122f is discussed in more detail below.

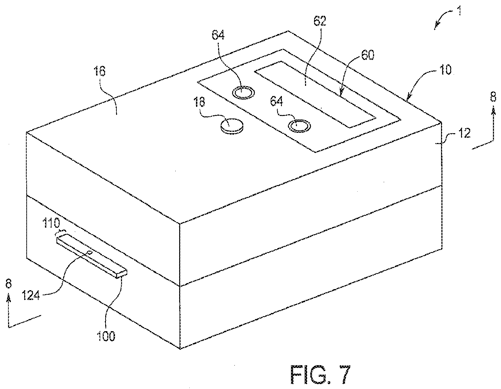

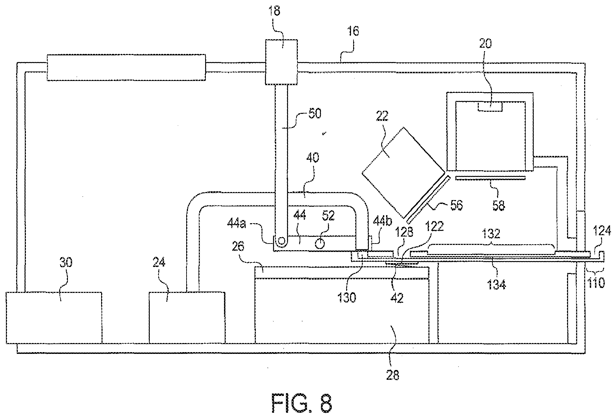

FIG. 7 illustrates a perspective view of device 10 after test card 100 has been placed in slot 14, and FIG. 8 illustrates a cross-sectional view thereof. In the illustrated embodiment, test card 100 is inserted so that an inlet portion 110 of test card 100 including inlet port 124 is located outside of housing 12 when test card 100 is fully inserted into slot 14. In this embodiment, a user can place test card 100 into housing 12 and then inject a fluid sample into inlet port 124. In an alternative embodiment, test card 100 can be inserted into slot 14 after a fluid sample has been injected into inlet port 124. In another alternative embodiment, test card 100 can be inserted into slot 14 so that no portion of test card 100 lies outside of housing 12.

As illustrated, placement of test card 100 into slot 14 aligns several of the elements of device 10 with several of the elements of test card 100. For example, placement of test card 100 into slot 14 aligns camera imaging device 20 and light source 22 of device 10 with analysis port 132 on an upper surface 104 of test card 100 (and with reaction zone 166 within analysis port 132), pneumatic tube 40 of fluid actuation source 24 with outlet port 130 on the upper surface 104 of test card 100, and electrical contacts 42 of electrical contact device 26 with electrical contacts 122 of a printed circuit layer on the bottom surface 102 of test card 100.

In the illustrated embodiment, fluid actuation source 24 includes a pneumatic tube 40 that is sealed against outlet port 130 of test card 100 by block 44 when test card 100 is inserted into slot 14. Vacuum source 24 is configured to apply a negative pneumatic pressure or vacuum to outlet port 130 via pneumatic tube 40, which causes the fluid sample inserted into inlet port 124 to be pulled from mixing chamber 126 through fluid microchannel 134 towards outlet port 130.

For vacuum source 24 to operate effectively, pneumatic tube 40 must be completely sealed against outlet port 130. In an embodiment, pneumatic tube 40 can be sealed against outlet port 130, for example, via a rubber gasket at the end of pneumatic tube 40. In another embodiment, pneumatic tube 40 can be sealed against block 44, and block 44 can be sealed against outlet port 130, for example, via a rubber gasket at the end of an aperture in block 44.

In the illustrated embodiment, button 18 protruding from the top surface 16 of housing 12 assists in sealing pneumatic tube 40 against outlet port 130 to align fluid actuation source 24 with outlet port 130. As illustrated, button 18 is attached to a first side 44a of block 44 via rod 50, and pneumatic tube 40 is attached to a second side 44b of block 44. Block 44 is configured to pivot about pivot point 52. By pushing button 18 downward at first side 44a, second side 44b with pneumatic tube 40 is rotated upward, which allows outlet port 130 of test card 100 to slide underneath second side 44b. When button 18 is released, second side 44b rotates downward so that pneumatic tube 40 is sealed against outlet port 130. In an embodiment, button 18 is biased upward, for example by a spring force, so that block 44 is biased to the configuration shown in FIG. 8.

Once pneumatic tube 40 is sealed against outlet port 130, a negative pneumatic force can be applied to outlet port 130 from fluid actuation source 24. When the negative pneumatic force is applied, the fluid sample previously injected into inlet port 124 is pulled through fluid microchannel 134 towards outlet port 130. The fluid sample however is not pulled into pneumatic tube 40 due to the presence of capture port 128 between inlet port 124 and outlet port 130. Capture port 128 allows fluid to build up before it can reach outlet port 130 and/or pneumatic tube 40, which keeps device 10 sterile and protects the integrity of diagnostic system 1.

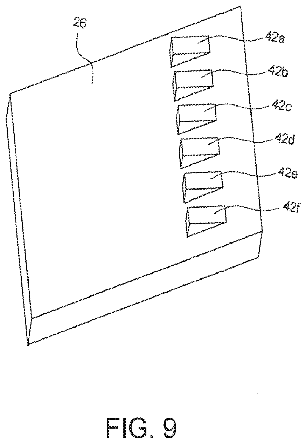

As illustrated in FIG. 8, test card 100 is dimensioned so that electrical contacts 122 of test card 100 are placed in electrical contact with electrical contacts 42 of electrical contact device 26 when test card 100 is fully inserted into slot 14 so that pneumatic tube 40 is sealed against outlet port 130. FIG. 9 illustrates electrical contact device 26 in more detail. As illustrated, electrical contact device 26 includes a plurality of electrical contacts 42a, 42b, 42c, 42d, 42e, 42f that are configured to align with the plurality of electrical contacts 122a, 122b, 122c, 122d, 122e, 122f of test card 100, so that each of electrical contacts 42 of electrical contact device 26 of device 10 is aligned with a separate electrical contact 122 of test card 100. In the illustrated embodiment, electrical contact 42a makes electrical contact with electrical contact 122a, electrical contact 42b makes electrical contact with electrical contact 122b, electrical contact 42c makes electrical contact with electrical contact 122c, electrical contact 42d makes electrical contact with electrical contact 122d, electrical contact 42e makes electrical contact with electrical contact 122e, and electrical contact 42f makes electrical contact with electrical contact 122f. By applying a current to each of electrical contacts 42a, 42b, 42c, 42d, 42e, 42f individually, the electrodes on test card 100 can be controlled independently of each other, and measurements can be taken from each electrode. Those of ordinary skill in the art will recognize that more or less electrical contacts can be used on assay device 10 and/or test card 100 depending on the number of electrodes on test card 100 that assay device 10 is required to control.

In the illustrated embodiments, electrical contacts 42 protrude upwardly at an angle from electrical contact device 26 and are configured to retract towards electrical contact device 26 as they make contact with test card 100. FIGS. 2 and 9 illustrate electrical contacts 42 before insertion of test card 100, where electrical contacts 42 are fully extended from electrical contact device 26. FIG. 8 illustrates electrical contacts 42 after insertion of test card 100, where the electrical contacts 42 have been pushed towards or into electrical contact device 26 by test card 100. By forming electrical contacts 42 as illustrated, it can be ensured that electrical contact is made between electrical contacts 42 of electrical contact device 26 and electrical contacts 122 of test card 100. Although the illustrated electrical contacts 42 are shown to have a triangular shape, those of ordinary skill in the art will recognize that other shapes and configurations are possible.

With the electrical contacts 42 and 122 aligned, device 10 can perform several functions. Before beginning an analysis, controller 28 can ensure that the fluid sample has been properly pulled through microchannel 134 and into target zone 166. By applying a current to electrical contact 42a aligned with electrical contact 122a and/or electrical contact 42f aligned with electrical contact 122f, controller 28 can activate the capacitor of first fluid detection zone 150 and measure the corresponding capacitance to ensure that the fluid sample has flowed through first fluid detection zone 150. Likewise, by applying a current to electrical contact 42c aligned with electrical contact 122c and/or electrical contact 42d aligned with electrical contact 142d, controller 28 can activate the capacitor of second fluid detection zone 154 and measure the corresponding capacitance to ensure that the fluid sample has flowed through second fluid detection zone 154. If fluid is located within both first fluid detection zone 150 and second fluid detection zone 154, controller 28 can determine that fluid from the fluid sample is located within target zone 166.

Once it is determined that fluid is located within target zone 166 of microchannel 134, controller 28 can apply a current to electrical contact 42b aligned with electrical contact 122b and to electrical contact 42e aligned with electrical contact 122e to heat the fluid sample within target zone 166 and cause a PCR to begin. During the PCR, current is applied to the electrodes 162a and 162b located adjacent to target zone 166 to raise the temperature of the fluid sample located within target zone 166. The desired temperature for different PCR's can vary. The temperature within target zone 166 can be monitored by a temperature sensor located within assay device 10 or test card 100 at a location adjacent to target zone 166, and controller 28 can receive feedback from the temperature sensor and adjust the current applied to electrical contact 42b and/or electrical contact 42e based on the feedback to maintain a desired temperature for the PCR.

Camera imaging device 20 is configured to record a series of still images of the fluid sample within target zone 166 during the PCR and/or a video of the fluid sample within target zone 166 during the PCR. In an embodiment, camera imaging system 20 includes a high sensitivity and dynamic range complementary metal-oxide semiconductor (CMOS) camera sensor which allows for general imaging of a PCR within target zone 166 of fluid microchannel 134 of test card 100. Camera imaging device 20 allows the PCR to be monitored in real time, by taking still and/or video images of the PCR within target zone 166 of fluid microchannel 134 over a period of time.

In the illustrated embodiment, light source 22 is configured to project a fluorescent excitation light on target zone 166 of fluid microchannel 134 while the PCR takes place, and while camera imaging device 20 takes still and/or video images of the PCR. When the PCR is illuminated with a fluorescent excitation light, controller 28 can make fluorescence measurements based on the images taken by camera imaging device 20, which can be analyzed by controller 28 to determine whether the fluid sample tests positive or negative for a particular bacteria or virus. Camera imaging device 20 also enables a variety of optical measurements to be taken in addition to fluorescence measurements, for example, turbidity and object detection measurements.

As illustrated, device 10 can include an excitation optical filter 56 located beneath light source 22, and an emission optical filter 58 located beneath camera imaging system 20. In an embodiment, excitation optical filter 56 functions to ensure only light of a wavelength below a cut off frequency of 500 nanometers is allowed to be incident to the test card 100 (short pass filter), and emission optical filter 58 functions to ensure only light greater than a cut off frequency 500 nanometers is allowed to be incident to camera imaging device 20 (long pass filter). These values can change depending on the emission spectra of a specific fluorescent reaction. In addition, by utilizing a long pass emission filter, a fluorescent reaction which has two emission peaks greater than the cut off frequency of the emission filter can be detected using a color camera sensor using a Bayer color filter.

An example embodiment of how controller 28 can analyze a fluid sample is described in more detail in U.S. Application No. 15/185,714, entitled "Camera Imaging System for a Fluid Sample Assay and Method of Using Same", filed concurrently herewith under Attorney Docket No. 1958928-00008. Those of ordinary skill in the art will recognize other analyses that can be performed using camera imaging device 20.

Device 10 is configured to accept a plurality of different types of test cards 100 intended for different types of assays. For example, a first type of test card can relate to a PCR, while a second type of test card can relate to flow cytometry. Assay device 10 is configured to accept different test cards 100, recognize the type of test card 100 at the time of insertion into slot 14, and run the appropriate assay for the test card without specific instructions by the user.

In an embodiment, each test card 100 includes a code, for example, a QR code or barcode, to identify the type of assay that the test card 100 is intended for. Assay device 10 can include a corresponding code reader that is positioned to align with the code on test card 100 when test card 10 is inserted into slot 14. In another embodiment, device 10 can include a user interface 60 including a display 62 and buttons 64. A user can enter a code written on test card 100 into user interface 60, and device 10 can recognize the code and run the appropriate test.

Each of the different types of test cards includes electrical contacts 122 in the same locations, although the electrical contacts can lead to different electrodes configured for different purposes. For example, in the embodiment illustrated in FIGS. 5 and 6, test card 100 has six electrical contacts corresponding to a target zone 166 and two fluid detection zones 150, 154. In another embodiment, for example, test card 100a can include six electrical contacts corresponding to three different target zones 166, or can include more or less than six electrical contacts corresponding to a target zone and different types of sensors such as capacitance and temperature sensors. By reading the code on each test card 100, controller 28 can determine the current to apply to each of electrical contacts 122 via electrical contacts 42 to run the assay desired by the user based on the card inserted.

For example, if test card 100 of FIGS. 3 to 5 is inserted into slot 14, controller 28 can read the code on test card 100 and determine that a single PCR is intended to be run within a single target zone 166. Controller 28 will therefore apply the desired current to electrical contacts 122c and 122d via electrical contacts 42c and 42d to cause the PCR to occur within target zone 166.

If an alternative embodiment of a test card 100a is instead inserted into slot 14, controller 28 can read the code on test card 100a and determine, for example, that a flow cytometry assay or an ELISA is intended to be run. Flow cytometry and ELISA tests do not require the fluid in target zone 166 to be heated to multiply molecules by diffusion, so the electrodes can be used for other purposes or omitted completely.

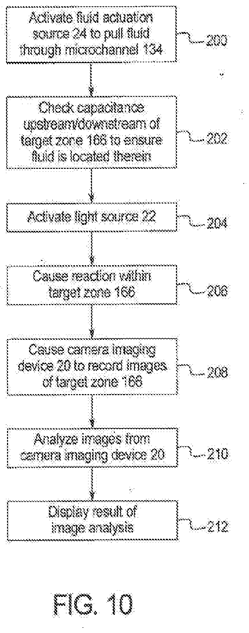

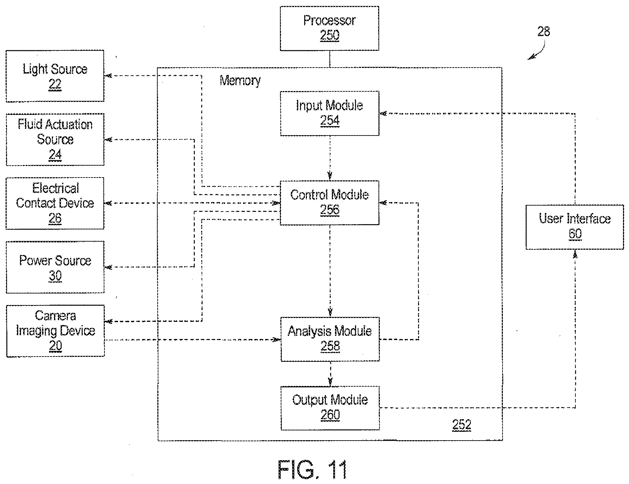

FIG. 10 illustrates an example embodiment of a control method that can be used by controller 28 to perform and analyze a reaction as described herein, and FIG. 11 illustrates an example embodiment of a controller 28 that can perform the method of FIG. 10. As illustrated, controller 28 can include a processor 250 and a memory 252, which can include a non-transitory computer readable medium. Memory 252 can include, for example, an input module 254, a control module 256, an analysis module 258, and an output module 260. Processor 250 can run the modules 252, 254, 256, 258 in accordance with instructions stored on memory 252. The broken lines in FIG. 8 illustrate the electrical connections between the modules 252, 254, 256, 258 of controller 28 and various elements of device 10. It should be understood by those of ordinary skill in the art that the illustrated modules and/or additional modules can be connected to the elements shown and/or additional elements.

The process begins by loading a test card 100 and/or a fluid sample into device 10. The fluid sample can be mixed with a reagent before injection into test card 100 and/or device 10, or can be mixed with a reagent within mixing chamber 26 of test card 100. In an embodiment, the reagent includes a PCR inhibitor-resistant polymerase along with a specific mixture of reverse transcriptase (in the case of RNA targets) and surfactants/dispersants to allow for rapid sample dispersion and lysing. In an embodiment, the reagent mix can include, for example, oligonucleotide primers, dNTP's DNA polymerase and other chemicals to assist the PCR. It is important to have a correct ratio of fluid sample to final PCR volume, because if the correct ratio is not maintained, the PCR will take too long or fail. In an embodiment, a PCR inhibitor resistant polymerase can be generated from mutant TAq.

Using user interface 60, a user can begin the reaction. As explained above, device 10 can indicate what type of test is to be run by reading a code on the test card. Alternatively, a user can choose an analysis to run on the fluid sample using the display 62 and buttons 64 of user interface 60. In an embodiment, a user can cycle through a plurality of tests on display 62 using buttons 64 and choose one or more test to run. The plurality of tests can include, for example, a PCR analysis, a cytometry analysis and/or an enzyme-linked immunosorbent assay (ELISA) analysis.

Input module 254 is configured to receive the user inputs inputted into user interface 60 and communicate the user inputs to control module 256. Input module 254 can also receive additional information via user interface 60 and/or by the preprogramming of controller 28, for example, (i) real-time PCR crossover threshold value information; (ii) maximum fluorescence information; and (iii) melting curve inflection temperature information.

Once a test card 100 and/or fluid sample has been loaded into device 10, control module 256 of controller 28 begins the control method at step 200 by causing fluid actuation source 26 to pull fluid through microchannel 134. In the illustrated embodiment, fluid actuation source 26 applies a negative pneumatic force to outlet port 130 via pneumatic tube 40 to pull fluid through microchannel 134. In an alternative embodiment, fluid actuation source can include one or more other type of pump in fluid communication with microchannel 134.

After fluid actuation source 26 has been activated, but before any reaction within target zone 166 begins, control module 256 at step 202 can verify that fluid is located within target zone 166 by monitoring the capacitance of microchannel 134 at one or more locations upstream and/or downstream of target zone 166 via electrodes 160 and 164. If fluid is detected in microchannel 134 upstream and downstream of target zone 166 by electrodes 160 and 164, control module 256 can verify that fluid is located within target zone 166, activate light source 22 at step 204, and begin a reaction at step 206.

If a PCR is being run, control module 256 begins the reaction at step 206 by causing power source 30 to send a current to electrodes 162a and 162b located adjacent to target zone 166 via electrical contact device 26 to cause the fluid within target zone 166 to be heated. As the fluid sample is heated, the nucleic acid molecules in the fluid sample multiply by diffusion, as explained in more detail in U.S. Application No. 15/185,714, entitled "Camera Imaging System for a Fluid Sample Assay and Method of Using Same", filed concurrently herewith under Attorney Docket No. 1958928-00008.

At the same time that the fluid sample is being heated within target zone 166 so that the nucleic acid molecules multiply by diffusion, control module 256 at step 208 can cause camera imaging device 20 to record a plurality of images of the reaction within target zone 166 through analysis port 132. The plurality of images can then be sent to analysis module 208 for analysis at step 210. In an embodiment, test card 100 includes a transparent material that allows images to be taken of target zone 166 of fluid microchannel 134 even though a layer of polymer material is located between camera imaging device 20 and fluid microchannel 134.

At step 210, analysis module 258 analyzes the images taken by camera imaging device 20 to determine whether the fluid sample tests positive or negative for a bacteria or virus. The type of analysis performed by analysis module at step 210 will depend on the type of assay being run on the fluid sample.

If the assay being run on the fluid sample is a PCR, then analysis module 258 can analyze the images, for example, by measuring fluorescence as nucleic molecules multiply by diffusion.

If the assay being run on the fluid sample is a flow cytometry analysis, then analysis module 258 can also analyze the images by measuring fluorescence. The cytometry analysis can differ from the PCR analysis, for example, because the fluid in target zone 166 does not need to be heated to multiply molecules by diffusion, so step 206 can be skipped. With a cytometry analysis, analysis module 258 can analyze the fluid sample within target zone 166, for example, by analyzing cell size, cell count, cell morphology (shape and structure), cell cycle phase, DNA content, and the existence or absence of specific proteins on cell surfaces. In an embodiment, analysis module 258 can also measure droplets in the case of droplet based reactions.

If the assay being run on the fluid sample is an ELISA analysis, then again the fluid in target zone 166 does not need to be heated to multiply molecules by diffusion, so step 206 can be skipped. With an ELISA analysis, analysis module 258 can analyze the fluid sample within target zone 166, for example, by measuring the concentration of an analyte in the fluid sample using a colormetric analysis. In an embodiment, a colorimetric analysis can include measuring the color intensities between target zones to determine the transmittance of light from each reaction chamber on the test card and determine the relative concentration of a specific analyte in each reaction chamber.

At step 212, analysis module 258 determines based on the analysis whether the fluid sample has tested positive or negative for a bacteria or virus. In an embodiment, display 62 can be configured to display a result of the analysis of analysis module 258 of controller 28. It is contemplated for the result to be a simple "positive" or "negative" result for the assay, so that device 10 can be quickly and easily used without the need for specialized training. User interface 60 is designed so that a user with minimal training can understand how to use both the device 10 and test cards 100. In an embodiment, analysis module can also display viral or bacterial load and the raw test data. In the case of a PCR reaction this includes amplification curves, melting curves, melting temperatures, fluorescence crossover cycle, etc.

Power source 30 is configured to provide power to all of the electrical components of device 10, for example, user interface 60, camera imaging device 20, light source 22, fluid actuation source 24, electrical contact device 26 and controller 28. In an embodiment, power source 30 includes a rechargeable or replaceable battery. In another embodiment, power source 30 can be plugged into a wall outlet to provide power or to be recharged by the wall outlet. Preferably, power source 30 is configured to store power so that device 10 can be used when there is no external power source present.

## Claims

1. A device (1) for performing an assay on a fluid sample, the device comprising:
a slot (14) configured to receive a test card (100), the test card including a microchannel (134) having a target zone (162, 166), a first fluid detection zone (150) that is upstream of the target zone (162, 166), and a second fluid detection zone (154) that is downstream of the target zone (162, 166); and
a vacuum source (24) configured to align with an outlet port (130) of the test card (100) when the test card is received within the slot;
wherein the device (1) includes:
an electrical contact device (26) having at least three pairs of electrical contacts (42) that are configured to contact corresponding electrical contacts (122) of the test card when the test card is received within the slot,
wherein a first pair of the electrical contacts is configured to provide an electrical current for heating the target zone (162, 166) of the microchannel (134) after the test card (100) is received within the slot (14), a second pair of the electrical contacts is configured for electrically connecting to a first capacitive sensor (160) of the test card (100) upstream of the target zone (162, 166) for detecting when the fluid sample is present within the first fluid detection zone (150) after the test card (100) is received within the slot (14), and a third pair of the electrical contacts are configured for electrically connecting to a second capacitive sensor (164) of the test card (100) downstream of the target zone (162, 166) for detecting when the fluid sample is present within the second fluid detection zone (154) after the test card (100) is received within the slot (14);
a camera imaging device (20) configured to align with the microchannel (134) of the test card (100) when the test card is received within the slot; and
a controller (28) configured to:
(i) cause the vacuum source (24) to pull the fluid sample through the microchannel (134) of the test card when the test card is received within the slot;
(ii) cause the electrical contact device (26) to apply current to the second pair and the third pair of the electrical contacts to activate the capacitive sensors (160, 164) and perform at least one capacitance measurement for each of the capacitive sensors (160, 164) to detect if the fluid sample is located within the target zone (162, 166) of the microchannel;
(iii) cause the electrical contact device (26) to apply current to the first pair of electrical contacts to heat the target zone (162, 166) if the fluid sample is located within the target zone (162, 166) of the microchannel; and
(iv) cause the camera imaging device (20) to record an image of the microchannel while the fluid sample is located within the microchannel.

2. The device (1) of Claim 1, wherein the controller is configured to cause the camera imaging device (20) to record the image during a polymerase chain reaction of the fluid sample when the target zone (162, 166) is heated while the fluid sample is located within the microchannel.

3. The device (1) of Claim 2, wherein the controller (28) is configured to analyze the image of the polymerase chain reaction and output a resulting analysis of the polymerase chain reaction.

4. The device (1) of Claims 2 or 3, wherein the test card (100) includes electrodes (166a, 162b) located at the target zone (166) of the microchannel and at least one electrical line placing the electrodes (166a, 162b) in electrical communication with the electrical contacts (122) that correspond to the first pair of the electrical contacts of the electrical contact device (26), and wherein a polymerase chain reaction is caused within the microchannel by the current being transmitted from the electrical contacts (122) to the electrodes (166a, 162b) via the at least one electrical line.

5. The device (1) of Claims 1 or 2, which includes a light source (22) configured to illuminate at least a portion of the microchannel when the test card is received within the slot.

6. The device (1) of any of Claims 1 to 5, wherein the image includes at least one of: (i) a plurality of still images recorded by the camera imaging device over a period of time; or (ii) a video image recorded by the camera imaging device over the period of time.

7. The device (1) of any of Claims 1 to 6, wherein the outlet port (130) is located on an upper surface (104) of the test card (100), and wherein the vacuum source (24) is configured to align with the outlet port on the upper surface of the test card.

8. The device (1) of Claim 7, which includes a button (18) configured to align the vacuum source (24) with the outlet port (130) on the upper surface (104) of the test card (100).

9. The device (1) of any of Claims 1 to 8, wherein the test card (100) includes an analysis port (132) located on an upper surface (104) of the test card (100), and wherein the camera imaging system is configured to align with the analysis port on the upper surface of the test card.

10. The device (1) of any of Claims 1 to 9, wherein the electrical contacts (122) are located on a bottom surface (102) of the test card (100), and wherein the at least three pairs of electrical contacts (42) of the electrical contact device (26) are configured to align with the electrical contacts (122) on the bottom surface of the test card.

11. The device (1) of any of Claims 1 to 10, which includes a current source configured to cause the current to be transmitted from the first pair of the electrical contacts of the electrical contact device (26) to the corresponding electrical contacts (122) of the test card to cause a polymerase chain reaction while the fluid sample is located within the target zone (162, 166).

12. The device (1) of any of Claims 1 to 11, which includes a user interface (60), wherein the controller (28) is configured to analyze the image and output the resulting analysis to the user interface to be displayed to a user of the device.

13. A method of using the device (1) of any of Claims 1 to 12, which includes:
injecting the fluid sample into an inlet port (124) of the test card (100);
placing the test card (100) into the device (1) of any of Claims 1 to 13 so that the electrical contacts (122) of the test card are placed into contact with the at least three pairs of electrical contacts (42) of the electrical contact device (26);
pulling the fluid sample through a microchannel (134) of the test card via the vacuum source (24);
applying the current to the second pair and the third pair of the electrical contacts to activate the capacitive sensors (160, 164) and perform at least one capacitance measurement of each of the capacitive sensors (160, 164);
determining if the fluid sample is located within the target zone (162, 166) of the microchannel based on the at least one capacitance measurement;
if the fluid sample is located within the target zone (162, 166) of the microchannel, heating the fluid sample to cause a polymerase chain reaction by transmitting a current from the first pair of the electrical contacts of the electrical contact device (26) to corresponding electrical contacts (122) that are electrically connected to the target zone (162, 166) of the test card; and
causing the camera imaging device (20) to record an image of the microchannel while the fluid sample is located within the microchannel.

## Patentansprüche

1. Eine Vorrichtung (1) zum Durchführen eines Assays an einer Fluidprobe, wobei die Vorrichtung Folgendes beinhaltet:
einen Schlitz (14), der dazu ausgelegt ist, eine Testkarte (100) aufzunehmen, wobei die Testkarte einen Mikrokanal (134) mit einem Zielbereich (162, 166), einem ersten Fluiddetektionsbereich (150), der stromaufwärts von dem Zielbereich (162, 166) liegt, und einem zweiten Fluiddetektionsbereich (154), der stromabwärts von dem Zielbereich (162, 166) liegt, umfasst; und
eine Unterdruckquelle (24), die dazu ausgelegt ist, mit einer Auslassöffnung (130) der Testkarte (100) ausgerichtet zu sein, wenn die Testkarte innerhalb des Schlitzes aufgenommen worden ist;
wobei die Vorrichtung (1) Folgendes beinhaltet:
eine elektrische Kontaktvorrichtung (26) mit mindestens drei Paaren von elektrischen Kontakten (42), die dazu ausgelegt sind, in Kontakt mit entsprechenden elektrischen Kontakten (122) der Testkarte zu kommen, wenn die Testkarte innerhalb des Schlitzes aufgenommen worden ist,
wobei ein erstes Paar der elektrischen Kontakte dazu ausgelegt ist, einen elektrischen Strom zum Erwärmen des Zielbereichs (162, 166) des Mikrokanals (134) bereitzustellen, nachdem die Testkarte (100) innerhalb des Schlitzes aufgenommen wurde (14), ein zweites Paar der elektrischen Kontakte zum elektrischen Anschließen an einen ersten kapazitiven Sensor (160) der Testkarte (100) stromaufwärts von dem Zielbereich (162, 166) ausgelegt ist, um zu detektieren, wann die Fluidprobe innerhalb des ersten Fluiddetektionsbereichs (150) vorhanden ist, nachdem die Testkarte (100) innerhalb des Schlitzes aufgenommen wurde (14), und ein drittes Paar der elektrischen Kontakte zum elektrischen Anschließen an einen zweiten kapazitiven Sensor (164) der Testkarte (100) stromabwärts von dem Zielbereich (162, 166) ausgelegt ist, um zu detektieren, wann die Fluidprobe innerhalb des zweiten Fluiddetektionsbereichs (154) vorhanden ist, nachdem die Testkarte (100) innerhalb des Schlitzes aufgenommen wurde (14);
eine Kamerabildgebungsvorrichtung (20), die dazu ausgelegt ist, mit dem Mikrokanal (134) der Testkarte (100) ausgerichtet zu sein, wenn die Testkarte innerhalb des Schlitzes aufgenommen worden ist; und
eine Steuereinrichtung (28), die zu Folgendem ausgelegt ist:
(i) Bewirken, dass die Unterdruckquelle (24) die Fluidprobe durch den Mikrokanal (134) der Testkarte zieht, wenn die Testkarte innerhalb des Schlitzes aufgenommen worden ist;
(ii) Bewirken, dass die elektrische Kontaktvorrichtung (26) Strom an das zweite Paar und das dritte Paar der elektrischen Kontakte anlegt, um die kapazitiven Sensoren (160, 164) zu aktivieren und mindestens eine Kapazitätsmessung für jeden der kapazitiven Sensoren (160, 164) durchzuführen, um zu detektieren, ob sich die Fluidprobe innerhalb des Zielbereichs (162, 166) des Mikrokanals befindet;
(iii) Bewirken, dass die elektrische Kontaktvorrichtung (26) Strom an das erste Paar von elektrischen Kontakten anlegt, um den Zielbereich (162, 166) zu erwärmen, wenn sich die Fluidprobe innerhalb des Zielbereichs (162, 166) des Mikrokanals befindet; und
(iv) Bewirken, dass die Kamerabildgebungsvorrichtung (20) ein Bild des Mikrokanals aufzeichnet, während sich die Fluidprobe innerhalb des Mikrokanals befindet.

2. Vorrichtung (1) gemäß Anspruch 1, wobei die Steuereinrichtung dazu ausgelegt ist, zu bewirken, dass die Kamerabildgebungsvorrichtung (20) das Bild während einer Polymerasekettenreaktion der Fluidprobe aufzeichnet, wenn der Zielbereich (162, 166) erwärmt wird, während sich die Fluidprobe innerhalb des Mikrokanals befindet.

3. Vorrichtung (1) gemäß Anspruch 2, wobei die Steuereinrichtung (28) dazu ausgelegt ist, das Bild der Polymerasekettenreaktion zu analysieren und eine resultierende Analyse der Polymerasekettenreaktion auszugeben.

4. Vorrichtung (1) gemäß Anspruch 2 oder 3, wobei die Testkarte (100) Elektroden (166a, 162b), die sich an dem Zielbereich (166) des Mikrokanals befinden, und mindestens eine elektrische Leitung umfasst, die die Elektroden (166a, 162b) in elektrische Verbindung mit den elektrischen Kontakten (122) bringt, die dem ersten Paar der elektrischen Kontakte der elektrischen Kontaktvorrichtung (26) entsprechen, und wobei durch den von den elektrischen Kontakten (122) über die mindestens eine elektrische Leitung zu den Elektroden (166a, 162b) übertragenen Strom eine Polymerasekettenreaktion innerhalb des Mikrokanals bewirkt wird.

5. Vorrichtung (1) gemäß Anspruch 1 oder 2, die eine Lichtquelle (22) umfasst, die dazu ausgelegt ist, mindestens einen Abschnitt des Mikrokanals zu beleuchten, wenn die Testkarte innerhalb des Schlitzes aufgenommen worden ist.

6. Vorrichtung (1) gemäß einem der Ansprüche 1 bis 5, wobei das Bild mindestens eines der Folgenden umfasst: (i) eine Vielzahl von Standbildern, die von der Kamerabildgebungsvorrichtung über einen Zeitraum aufgezeichnet wurden; oder (ii) ein Videobild, das von der Kamerabildgebungsvorrichtung über den Zeitraum aufgezeichnet wurde.

7. Vorrichtung (1) gemäß einem der Ansprüche 1 bis 6, wobei sich die Auslassöffnung (130) auf einer oberen Oberfläche (104) der Testkarte (100) befindet und wobei die Unterdruckquelle (24) dazu ausgelegt ist, mit der Auslassöffnung auf der oberen Oberfläche der Testkarte ausgerichtet zu werden.

8. Vorrichtung (1) gemäß Anspruch 7, die einen Knopf (18) umfasst, der dazu ausgelegt ist, die Unterdruckquelle (24) mit der Auslassöffnung (130) auf der oberen Oberfläche (104) der Testkarte (100) auszurichten.

9. Vorrichtung (1) gemäß einem der Ansprüche 1 bis 8, wobei die Testkarte (100) eine Analysenöffnung (132) umfasst, die sich auf einer oberen Oberfläche (104) der Testkarte (100) befindet, und wobei das Kamerabildgebungssystem dazu ausgelegt ist, mit der Analysenöffnung auf der oberen Oberfläche der Testkarte ausgerichtet zu sein.

10. Vorrichtung (1) gemäß einem der Ansprüche 1 bis 9, wobei sich die elektrischen Kontakte (122) auf einer unteren Oberfläche (102) der Testkarte (100) befinden und wobei die mindestens drei Paare von elektrischen Kontakten (42) der elektrischen Kontaktvorrichtung (26) dazu ausgelegt sind, mit den elektrischen Kontakten (122) auf der unteren Oberfläche der Testkarte ausgerichtet zu sein.

11. Vorrichtung (1) gemäß einem der Ansprüche 1 bis 10, die eine Stromquelle umfasst, die dazu ausgelegt ist, zu bewirken, dass der Strom von dem ersten Paar der elektrischen Kontakte der elektrischen Kontaktvorrichtung (26) zu den entsprechenden elektrischen Kontakten (122) der Testkarte übertragen wird, um eine Polymerasekettenreaktion zu bewirken, während sich die Fluidprobe innerhalb des Zielbereichs (162, 166) befindet.

12. Vorrichtung (1) gemäß einem der Ansprüche 1 bis 11, die eine Benutzerschnittstelle (60) umfasst, wobei die Steuereinrichtung (28) dazu ausgelegt ist, das Bild zu analysieren und die resultierende Analyse an die Benutzerschnittstelle auszugeben, um einem Benutzer der Vorrichtung angezeigt zu werden.

13. Ein Verfahren zum Verwenden der Vorrichtung (1) gemäß einem der Ansprüche 1 bis 12, das Folgendes umfasst:
Injizieren der Fluidprobe in eine Einlassöffnung (124) der Testkarte (100);
Platzieren der Testkarte (100) in der Vorrichtung (1) gemäß einem der Ansprüche 1 bis 13, sodass die elektrischen Kontakte (122) der Testkarte in Kontakt mit den mindestens drei Paaren von elektrischen Kontakten (42) der elektrischen Kontaktvorrichtung (26) platziert werden;
Ziehen der Fluidprobe mittels der Unterdruckquelle (24) durch einen Mikrokanal (134) der Testkarte;
Anlegen des Stroms an das zweite Paar und das dritte Paar der elektrischen Kontakte, um die kapazitiven Sensoren (160, 164) zu aktivieren und mindestens eine Kapazitätsmessung jedes der kapazitiven Sensoren (160, 164) durchzuführen;
Bestimmen auf Grundlage der mindestens einen Kapazitätsmessung, ob sich die Fluidprobe innerhalb des Zielbereichs (162, 166) des Mikrokanals befindet;
falls sich die Fluidprobe innerhalb des Zielbereichs (162, 166) des Mikrokanals befindet, Erwärmen der Fluidprobe, um eine Polymerasekettenreaktion zu bewirken, indem ein Strom von dem ersten Paar der elektrischen Kontakte der elektrischen Kontaktvorrichtung (26) zu entsprechenden elektrischen Kontakten (122), die elektrisch mit dem Zielbereich (162, 166) der Testkarte verbunden sind, übertragen wird; und
Bewirken, dass die Kamerabildgebungsvorrichtung (20) ein Bild des Mikrokanals aufzeichnet, während sich die Fluidprobe innerhalb des Mikrokanals befindet.

## Revendications

1. Un dispositif (1) pour effectuer un dosage sur un échantillon de fluide, le dispositif comprenant :
une fente (14) configurée pour recevoir une carte de test (100), la carte de test incluant un microcanal (134) ayant une zone cible (162, 166), une première zone de détection de fluide (150) qui se trouve en amont de la zone cible (162, 166), et une deuxième zone de détection de fluide (154) qui se trouve en aval de la zone cible (162, 166) ; et
une source de vide (24) configurée pour s'aligner avec un orifice de sortie (130) de la carte de test (100) quand la carte de test est reçue à l'intérieur de la fente ;
le dispositif (1) incluant :
un dispositif de contact électrique (26) ayant au moins trois paires de contacts électriques (42) qui sont configurés pour entrer en contact avec des contacts électriques correspondants (122) de la carte de test quand la carte de test est reçue à l'intérieur de la fente ;
dans lequel une première paire des contacts électriques est configurée pour fournir un courant électrique pour chauffer la zone cible (162, 166) du microcanal (134) après que la carte de test (100) est reçue à l'intérieur de la fente (14), une deuxième paire des contacts électriques est configurée pour se connecter électriquement à un premier capteur capacitif (160) de la carte de test (100) en amont de la zone cible (162, 166) pour détecter quand l'échantillon de fluide est présent à l'intérieur de la première zone de détection de fluide (150) après que la carte de test (100) est reçue à l'intérieur de la fente (14), et une troisième paire des contacts électriques est configurée pour se connecter électriquement à un deuxième capteur capacitif (164) de la carte de test (100) en aval de la zone cible (162, 166) pour détecter quand l'échantillon de fluide est présent à l'intérieur de la deuxième zone de détection de fluide (154) après que la carte de test (100) est reçue à l'intérieur de la fente (14) ;
un dispositif d'imagerie par caméra (20) configuré pour s'aligner avec le microcanal (134) de la carte de test (100) quand la carte de test est reçue à l'intérieur de la fente ; et
un organe de commande (28) configuré pour :
(i) amener la source de vide (24) à tirer l'échantillon de fluide à travers le microcanal (134) de la carte de test quand la carte de test est reçue à l'intérieur de la fente ;
(ii) amener le dispositif de contact électrique (26) à appliquer un courant à la deuxième paire et à la troisième paire des contacts électriques afin d'activer les capteurs capacitifs (160, 164) et d'effectuer au moins une mesure de capacité pour chacun des capteurs capacitifs (160, 164) afin de détecter si l'échantillon de fluide est situé à l'intérieur de la zone cible (162, 166) du microcanal ;
(iii) amener le dispositif de contact électrique (26) à appliquer un courant à la première paire de contacts électriques afin de chauffer la zone cible (162, 166) si l'échantillon de fluide est situé à l'intérieur de la zone cible (162, 166) du microcanal ; et
(iv) amener le dispositif d'imagerie par caméra (20) à enregistrer une image du microcanal alors que l'échantillon de fluide est situé à l'intérieur du microcanal.

2. Le dispositif (1) de la revendication 1, dans lequel l'organe de commande est configuré pour amener le dispositif d'imagerie par caméra (20) à enregistrer l'image pendant une réaction en chaîne par polymérase de l'échantillon de fluide quand la zone cible (162, 166) est chauffée alors que l'échantillon de fluide est situé à l'intérieur du microcanal.

3. Le dispositif (1) de la revendication 2, dans lequel l'organe de commande (28) est configuré pour analyser l'image de la réaction en chaîne par polymérase et produire en sortie une analyse résultante de la réaction en chaîne par polymérase.

4. Le dispositif (1) des revendications 2 ou 3, dans lequel la carte de test (100) inclut des électrodes (166a, 162b) situées au niveau de la zone cible (166) du microcanal et au moins une ligne électrique plaçant les électrodes (166a, 162b) en communication électrique avec les contacts électriques (122) qui correspondent à la première paire des contacts électriques du dispositif de contact électrique (26), et dans lequel une réaction en chaîne par polymérase est entraînée à l'intérieur du microcanal par le courant qui est transmis des contacts électriques (122) aux électrodes (166a, 162b) par l'intermédiaire de l'au moins une ligne électrique.

5. Le dispositif (1) des revendications 1 ou 2, qui inclut une source de lumière (22) configurée pour éclairer au moins une portion du microcanal quand la carte de test est reçue à l'intérieur de la fente.

6. Le dispositif (1) de n'importe lesquelles des revendications 1 à 5, dans lequel l'image inclut au moins un élément parmi : (i) une pluralité d'images fixes enregistrées par le dispositif d'imagerie par caméra au cours d'une période de temps ; ou (ii) une image vidéo enregistrée par le dispositif d'imagerie par caméra au cours de la période de temps.

7. Le dispositif (1) de n'importe lesquelles des revendications 1 à 6, dans lequel l'orifice de sortie (130) est situé sur une surface de dessus (104) de la carte de test (100), et dans lequel la source de vide (24) est configurée pour s'aligner avec l'orifice de sortie sur la surface de dessus de la carte de test.

8. Le dispositif (1) de la revendication 7, qui inclut un bouton (18) configuré pour aligner la source de vide (24) avec l'orifice de sortie (130) sur la surface de dessus (104) de la carte de test (100).

9. Le dispositif (1) de n'importe lesquelles des revendications 1 à 8, dans lequel la carte de test (100) inclut un orifice d'analyse (132) situé sur une surface de dessus (104) de la carte de test (100), et dans lequel le système d'imagerie par caméra est configuré pour s'aligner avec l'orifice d'analyse sur surface de dessus de la carte de test.

10. Le dispositif (1) de n'importe lesquelles des revendications 1 à 9, dans lequel les contacts électriques (122) sont situés sur une surface de dessous (102) de la carte de test (100), et dans lequel les au moins trois paires de contacts électriques (42) du dispositif de contact électrique (26) sont configurées pour s'aligner avec les contacts électriques (122) sur la surface de dessous de la carte de test.

11. Le dispositif (1) de n'importe lesquelles des revendications 1 à 10, qui inclut une source de courant configurée pour amener le courant à être transmis de la première paire des contacts électriques du dispositif de contact électrique (26) aux contacts électriques correspondants (122) de la carte de test afin d'entraîner une réaction en chaîne par polymérase alors que l'échantillon de fluide est situé à l'intérieur de la zone cible (162, 166).

12. Le dispositif (1) de n'importe lesquelles des revendications 1 à 11, qui inclut une interface utilisateur (60), dans lequel l'organe de commande (28) est configuré pour analyser l'image et produire en sortie l'analyse résultante vers l'interface utilisateur pour qu'elle soit affichée à destination d'un utilisateur du dispositif.

13. Un procédé d'utilisation du dispositif (1) de n'importe lesquelles des revendications 1 à 12, qui inclut :
le fait d'injecter l'échantillon de fluide dans un orifice d'entrée (124) de la carte de test (100) ;
le fait de placer la carte de test (100) dans le dispositif (1) de n'importe lesquelles des revendications 1 à 13 de sorte que les contacts électriques (122) de la carte de test soient placés en contact avec les au moins trois paires de contacts électriques (42) du dispositif de contact électrique (26) ;
le fait de tirer l'échantillon de fluide à travers un microcanal (134) de la carte de test par l'intermédiaire de la source de vide (24) ;
le fait d'appliquer le courant à la deuxième paire et à la troisième paire des contacts électriques afin d'activer les capteurs capacitifs (160, 164) et d'effectuer au moins une mesure de capacité de chacun des capteurs capacitifs (160, 164) ;
le fait de déterminer si l'échantillon de fluide est situé à l'intérieur de la zone cible (162, 166) du microcanal sur la base de l'au moins une mesure de capacité ;
si l'échantillon de fluide est situé à l'intérieur de la zone cible (162, 166) du microcanal, le fait de chauffer l'échantillon de fluide afin d'entraîner une réaction en chaîne par polymérase par transmission d'un courant de la première paire des contacts électriques du dispositif de contact électrique (26) aux contacts électriques correspondants (122) qui sont connectés électriquement à la zone cible (162, 166) de la carte de test ; et
le fait d'amener le dispositif d'imagerie par caméra (20) à enregistrer une image du microcanal alors que l'échantillon de fluide est situé à l'intérieur du microcanal.
